# EUROPÄISCHE PATENTSCHRIFT

(11) **EP 1 714 670 B1**
(45) Veröffentlichungstag und Bekanntmachung des Hinweises auf die Patenterteilung: **12.09.2007**
(21) Anmeldenummer: 06006092.8
(22) Anmeldetag: 24.03.2006
(51) Int. Cl.: A61N 1/00

(54) **Herzschrittmacher**
Pacemaker
Stimulateur cardiaque

(30) Priorität: 22.04.2005 DE 102005020071
(43) Veröffentlichungstag der Anmeldung: 25.10.2006
(73) Patentinhaber: BIOTRONIK CRM Patent AG, 6341 Baar (CH)
(72) Erfinder: Neumann, Andreas, 10969 Berlin (DE); Lewalter, Thorsten, Dr., 53127 Bonn (DE); Philipp, Jens, Dr., 10555 Berlin (DE)
(74) Vertreter: Lindner-Vogt, Karin L.

(56) Entgegenhaltungen:
- EP-A- 0 916 363
- EP-A- 1 139 477
- WO-A-90/02581
- US-A- 3 943 936
- US-A- 5 411 535

## Beschreibung

Die Erfindung betrifft einen Herzschrittmacher, der wenigstens zur Stimulation eines Atriums oder eines Ventrikels ausgebildet ist. Der Herzschrittmacher besitzt ein dichtes Gehäuse, in welchem eine Batterie und eine Schrittmachersteuerung, ein Stimulationsimpulsgenerator sowie eine Erfassungsstufe für Herzsignale angeordnet sind, die jeweils mit der Batterie verbunden sind.

In einer bevorzugten Variante betrifft die Erfindung jedoch auch einen Zweikammerschrittmacher, das heißt, einen Herzschrittmacher, der in der Lage ist, sowohl das Atrium als auch den Ventrikel eines Herzens, und zwar vorzugsweise das rechte Atrium und den rechten Ventrikel zu stimulieren.

Implantierbare Herzschrittmacher in ihrer grundsätzlich bekannten Form besitzen ein Gehäuse, in welchem eine Batterie angeordnet ist, die einen Betrieb des Schrittmachers über viele Jahre erlaubt. Die Batterie versorgt eine Schrittmachersteuerung, die in der Regel mit einer Erfassungsstufe für Herzsignale sowie mit einem Stimulationsimpulsgenerator verbunden ist. Sowohl der Stimulationsimpulsgenerator als auch die Erfassungsstufe für Herzsignale sind ihrerseits wiederum mit Anschlüssen für eine oder mehrere Elektrodenleitungen verbunden, die es erlauben, vom Stimulationsimpulsgenerator erzeugte Stimulationsimpulse an das Muskelgewebe des Herzens (Myokard) abzugeben oder elektrische Potentiale im Herz zu erfassen und zu verarbeiten.

Die verschiedenen Betriebsweisen und Varianten solcher Herzschrittmacher sind grundsätzlich bekannt und brauchen hier nicht weiter erläutert zu werden. Die Betriebsweise eines Herzschrittmachers wird durch einen standardisierten Buchstabencode charakterisiert. Im Rahmen dieser standardisierten Nomenklatur ist ein DDD-Schrittmacher ein Schrittmacher, der Stimulationsimpulse sowohl an den Ventrikels als auch an das Atrium abgeben kann, der außerdem elektrische Potentiale sowohl im Ventrikel als auch im Atrium erfassen kann und der schließlich unter anderem in einem Demand-Modus zu betreiben ist, in dem Stimulationsimpulse an den Ventrikel oder an das Atrium nur dann abgegeben werden, wenn dies erforderlich ist, das heißt, wenn innerhalb eines ventrikulären oder eines atrialen Escape-Intervalls kein mit einer natürlichen Kontraktion des Atriums oder des Ventrikels einhergehendes elektrisches Potential erfasst wird. Wird während der Dauer des Escape-Intervalls eine natürliche Herzkontraktion erfasst, wird die Abgabe eines entsprechenden Stimulationsimpulses unterdrückt (inhibiert). Ein Schrittmacher, der in der Lage ist, das Atrium im Demand-Modus zu stimulieren wird als AAI-Schrittmacher bezeichnet. Der erste Buchstabe beschreibt die Fähigkeit des Schrittmachers, Stimulationsimpulse an das Atrium abzugeben, der zweite Buchstabe beschreibt die Fähigkeit des Schrittmachers, atriale Herzaktionen zu erfassen und der dritte Buchstabe beschreibt die Fähigkeit des Schrittmachers, die Abgabe eines atrialen Stimulationsimpulses zu unterdrücken, falls innerhalb eines gesetzten Intervalls, üblicherweise als Escape-Intervall bezeichnet, eine natürliche Herzaktion erfasst wird. Entsprechend wird ein Schrittmacher, der den Ventrikel im Demand-Modus stimulieren kann, als VVI-Herzschrittmacher bezeichnet.

Bekannte Herzschrittmacher werden typischerweise unterhalb des Schlüsselbeins eines Patienten implantiert. Die Verbindung zum Herzen erfolgt über flexible Elektrodenleitungen, die in der jeweiligen Herzkammer (Atrium oder Ventrikel) enden und dort mit Elektroden versehen sind. Schrittmacherseitig sind die Elektrodenleitungen mit genormten Steckern versehen, die zum Herstellen der gewünschten elektrischen Verbindung ebenfalls genormte Steckbuchsen des Herzschrittmachers eingreifen. Diese Steckbuchsen des Schrittmachers sind üblicherweise in einem aus elektrisch isolierendem Kunststoff gefertigten, sogenannten Header des Schrittmachers angeordnet, der über eine sogenannte Durchführung elektrisch mit der oder den Erfassungsstufen und dem oder den Stimulationsimpulsgeneratoren im Inneren eines dichten Metallgehäuses des Schrittmachers elektrisch verbunden ist. Dieses Metallgehäuse beherbergt außerdem eine Batterie zum Betreiben des Schrittmachers sowie eine Steuerelektronik. Die Stimulationsimpulsgeneratoren umfassen üblicherweise Kondensatoren, in denen vor Abgabe eines Stimulationsimpulses die Energie für den Stimulationsimpuls gespeichert wird. Das Laden der Kondensatoren erfolgt mit Hilfe einer geeigneten Ladestufe, die ihre Energie aus der Batterie des Schrittmachers bezieht. Üblicherweise wird ein großer Teil dieses Metallgehäuses, wenn nicht gar der größte Teil des Metallgehäuses von der Batterie des Schrittmachers eingenommen.

Aus US 3,943,936 ist ein Herzschrittmacher bekannt, der nicht länger als 30 mm ist und einen Durchmesser von nicht mehr als 10 mm aufweist und der beispielsweise im rechten Ventrikel eines Herzens platziert werden kann.

Aus US 5,411,535 ist ein Herzschrittmacher bekannt, der ein Hauptgehäuse besitzt, welches an üblicher Stelle unterhalb des Schlüsselbeins zu implantieren ist und welches drahtlos mit wenigstens einer Elektrode im Ventrikel korrespondiert. Die drahtlos mit dem Hauptgehäuse verbundenen Elektroden stellen keine autarken Herzschrittmacher dar.

Die hier vorgestellte Erfindung hat das Ziel, einen Herzschrittmacher anzugeben, der manche der Beschränkungen der hier vorgestellten Standardanordnung eines Schrittmachers vermeidet und so neue Anwendungsfelder für die Schrittmachertherapie erschließt. Die Erfindung wird in den Ansprüchen 1 und 22 definiert.

Erfindungsgemäß wird dieses Ziel mit einem Herzschrittmacher der eingangs genannten Art erzielt, dessen Gehäuse nicht wie bei bekannten Schrittmachern relativ flach, sondern längsgestreckt ist und beispielsweise stabförmig oder zigarrenförmig ausgebildet ist. Die Querschnittfläche des Gehäuses beträgt weniger als 100 mm². Dabei ist der maximale Durchmesser des Gehäuses 12 mm, so dass sich eine Querschnittsform ergibt, deren Durchmesser in verschiedene Richtungen nicht um einen größeren Faktor von beispielsweise mehr als 2 voneinander unterscheiden. Die Länge des Gehäuses beträgt maximal 70 mm.

Eine volle Funktionalität des Herzschrittmachers bei gleichzeitig ausreichender Batterielebensdauer ist gewährleistet, wenn das Gehäusevolumen weniger als 7 cm³ beträgt. Das größte Querschnittsmaß des Gehäuses von maximal 12 mm erlaubt so eine transvenöse Implantation. Besonders vorteilhaft ist es, wenn das Gehäuse einen wenigstens annähernd runden Querschnitt besitzt. Alternative, ebenfalls vorteilhafte Querschnittsformen wären leicht abgeflachte, kreisähnliche Querschnitte, beispielsweise ovale oder elliptische Querschnitte. Auch Polygone mit abgerundeten Ecken stellen eine geeignete Querschnittsform dar.

Weiterhin ist es vorteilhaft, wenn der Herzschrittmacher über den größten Teil seiner Länge dieselbe Querschnittsform besitzt und in besonders vorteilhafter Weise zylindrisch geformt ist.

In einer bevorzugten Ausführungsvariante ist die Längsachse des Herzschrittmachers gerade, sie kann aber auch schwach gekrümmt sein. Der Krümmungsradius beträgt dabei vorzugsweise ein Mehrfaches der Länge des Herzschrittmachers.

Das Gehäuse trägt wenigstens zwei Elektroden, die jeweils eine nach außen gerichtete, elektrisch leitende Oberfläche aufweisen und als Stimulationselektroden ausgebildet sowie wenigstens zeitweise mit dem Stimulationsimpulsgenerator elektrisch über eine im Inneren des Gehäuses angeordnete elektrische Verbindung und ein elektrisches Schaltelement verbunden sind. Als Elektroden werden in diesem Zusammenhang jene elektrisch leitenden Bauelemente bezeichnet, über die beispielsweise Stimulationsimpulse abgegeben oder auch elektrische Potentiale erfasst werden können. Die Elektroden bilden somit mit ihrer elektrisch leitenden Oberfläche Pole für die Stimulation. Sie sind bei dem erfindungsgemäßen Herzschrittmacher in dessen Gehäuse integriert.

Ein derartiger Herzschrittmacher hat die Eigenschaft, beispielsweise in dem vom rechten Atrium aus zugänglichen Herzohr oder direkt im Ventrikel angeordnet werden zu können und aufgrund der auf seiner Oberfläche angeordneten Stimulationselektroden als autarkes Gerät wenigstens das Atrium oder den Ventrikel stimulieren zu können.

Von den Elektroden (Polen) ist vorzugsweise wenigstens eine erste Elektrode sehr kleinflächig - also beispielsweise punktförmig - und besitzt eine Oberfläche von weniger als 5 mm². Diese erste Elektrode ist vorzugsweise an einem Längsende des längsgestreckten Gehäuses angeordnet. Auch die zweite Elektrode kann kleinflächig sein und beispielsweise die gleiche Form besitzen, wie die erste Elektrode. Vorzugsweise sind beide Elektroden in unmittelbarer Nachbarschaft zueinander am selben Längsende des Gehäuses angeordnet.

Die zweite Elektrode kann aber auch von einem großflächigen Gehäuseteil des Gehäuses des Herzschrittmachers gebildet sein und bildet so einen indifferenten Pol, der quasi als Neutralelektrode wirkt.

In einer alternativen Ausführungsvariante sind die Elektroden als Ringelektroden ausgebildet, die um den Umfang des Gehäuses umlaufen. Auch in diesem Fall sind die Elektroden vorzugsweise einander dicht benachbart angeordnet. Auf diese Weise ist die genaue Ausrichtung des Herzschrittmachers nach Implantation beispielsweise im rechten Herzohr oder auch im Ventrikel relativ unkritisch.

Der Herzschrittmacher ist vorzugsweise als Einkammer-Demand-Schrittmacher ausgebildet (AAI oder VVI). Zu diesem Zweck dienen die Elektroden vorteilhafter Weise zusätzlich als Abfühlelektroden, die wenigstens zeitweise mit einer Erfassungsstufe im Inneren des Gehäuses zu verbinden sind.

Um die Funktionalität des an sich autarken Herzschrittmachers zu erweitern, ist es vorteilhaft, wenn der Herzschrittmacher einen Telemetriesender und einen Telemetrieempfänger besitzt, der mit der Schrittmachersteuerung verbunden ist und es erlaubt, Betriebsdaten des Herzschrittmachers sowie erfasste, physiologische Daten, beispielsweise intrakardiale Elektrokardiogramme, an ein externes Gerät zu übertragen und umgekehrt Programmier- und Steuerbefehle zu empfangen. Auch Betriebsparameter, beispielsweise wie eine jeweils zu wählende Stimulationsimpulsstärke können auf diese Weise mittels eines externen Gerätes ermittelt und an den Herzschrittmacher übertragen werden, so dass auch die Betriebsparameter dann für den weiteren, auch autarken Schrittmacherbetrieb in einem entsprechenden Speicher der Schrittmachersteuerung zu speichern sind.

Um mit einem im Herzen selbst implantierbaren Herzschrittmacher nicht nur das rechte Atrium, sondern beispielsweise auch den rechten oder linken Ventrikel stimulieren zu können, besitzt der Herzschrittmacher vorzugsweise eine drahtgebundene oder besser noch drahtlose Schnittstelle, die mit der Schrittmachersteuerung verbunden und ausgebildet ist, mit einer entsprechenden Schnittstelle eines zweiten Schrittmachers Daten bezüglich der Zeitpunkte in einer jeweiligen Herzkammer auftretender Ereignisse (natürliche oder stimulierte Kammerkontraktionen) auszutauschen. Auf diese Weise wird ein System aus zwei oder mehr Schrittmachern möglich, von denen einer im Atrium und einer im Ventrikel platziert werden kann und die zusammen die Funktionalität eines Zweikammerschrittmachers besitzen. In einem solchen System übermittelt der atriale Herzschrittmacher dem ventrikulären Herzschrittmacher die Zeitpunkte atrialer Ereignisse und vice versa. Somit kann die Steuerung des jeweils einen Herzschrittmachers auf Ereignisse in der jeweils anderen Herzkammer ansprechen, also beispielsweise der ventrikuläre Herzschrittmacher auf atriale Ereignisse, so dass eine an sich bekannte, atriumsynchrone Stimulation des Ventrikels mit physiologisch adäquater atrioventrikulärer Verzögerungszeit (AV-Intervall) möglich ist.

Mit mehr als zwei intrakardial implantierbaren Schrittmachern der erfindungsgemäßen Art ist beispielsweise auch eine Dreikammerstimulation im Rahmen einer Resychronisations-Therapie möglich.

Anstelle einer Zweikammerfunktion wie zuvor beschrieben mit zwei miteinander datenverbundenen Herzschrittmachern der erfindungsgemäßen Art zu verwirklichen, kann einem in einer Herzkammer zu platzierenden Herzschrittmacher auch eine Elektrodenleitung für eine jeweils andere Herzkammer angeschlossen sein. Diese Elektrodenleitung ist biegeweich und trägt wenigstens eine Stimulationselektrode an ihrem freien Ende oder in der Nähe ihres freien Endes. Ihre Länge ist so bemessen, dass sie entweder über den Coronar Sinus und gegebenenfalls eine davon abzweigende Lateralvene eine Stimulation des linken Ventrikels erlaubt oder durch das rechte Atrium hindurch in den rechten Ventrikel ragt. Bevorzugt ist eine Elektrodenleitung, die in den Coronar Sinus und in eine vom Coronar Sinus abzweigende Lateralvene einzuführen ist. Die Elektrodenleitung ist vorzugsweise dauerhaft an das Gehäuse des Herzschrittmachers angeschlossen und besitzt in jedem Fall einen elektrischen Leiter, der die Stimulationselektrode der Elektrodenleitung mit dem Stimulationsimpulsgenerator des Herzschrittmachers verbindet. Auf diese Weise wird der Herzschrittmacher zum Zweikammer-Schrittmacher, der für die Stimulation der Herzkammer, in der er im Anwendungsfall platziert ist, Elektroden auf seinem Gehäuse trägt und der zur Stimulation der anderen Herzkammer die biegeweiche Elektrodenleitung besitzt. Um eine Zweikammer-Stimulation zu ermöglichen, kann der Herzschrittmacher einen einzigen Stimulationsimpulsgenerator besitzen, der abwechselnd mit den atrialen Stimulationselektroden und den ventrikulären Stimulationselektroden zu verbinden ist. Es können auch zwei separate Stimulationsimpulsgeneratoren für das Atrium und den Ventrikel vorgesehen sein, die mit einer Leitung verbunden sind.

Bei einem Herzschrittmacher, der einen vorzugsweise kreisrunden Querschnitt besitzt und dessen Gehäuse vorzugsweise wenigstens über einen Teil seiner Länge zylindrisch geformt ist, kann in vorteilhafter- und das Gehäusevolumen bestmöglich ausnutzender Weise eine Batterie Verwendung finden, deren Querschnitt ebenfalls vorzugsweise rund ist. Dies erlaubt es in besonders vorteilhafter Weise, die Elektroden der Batterie in an sich bekannter Art und Weise zu wickeln. Das anzustrebende Batterievolumen beträgt zwischen 1 cm³ und 3 cm³. Die Batteriekapazität liegt vorzugsweise zwischen 0,25 und 0,75 Ah.

Das Gehäuse wird vorzugsweise von miteinander verschweißten Gehäuseelementen aus Metall gebildet. Wenigstens eine der Elektroden ist gegenüber dem verschweißten Metall-Gehäuse elektrisch isoliert. Von den Metall-Gehäuseelementen ist beispielsweise eines röhrenförmig mit geschlossenem Boden ausgeführt. In dieses Gehäuseelement können Batterie und Steuerelektronik eingesetzt werden. Dies erlaubt es in vorteilhafter Weise das Gehäuse aus beispielsweise drei Gehäuseelementen zusammenzusetzen, nämlich aus einem zentralen, röhrenförmigen Gehäuseelement sowie zwei Endkappen.

Die Stimulations- und/oder Abfühlelektroden können dann jeweils zwischen einer der Gehäusekappen und dem zentralen, röhrenförmigen Gehäuselimit angeordnet werden und sind an diesem Ort auch leicht zu montieren.

Alternativ können die Gehäuseelemente aus Keramik oder Kunststoff gefertigt sein und die Elektroden können von Metallringen gebildet sein.

Die Erfindung soll nun anhand eines Ausführungsbeispiels näher erläutert werden. Der Illustration des Ausführungsbeispiels dienen Figuren, von denen
- Fig. 1:: eine Außenansicht einer ersten Variante eines im rechten Herzohr oder im Ventrikel zu implantierenden Einkammer-Herzschrittmachers ist;
- Fig. 2:: eine perspektivische Explosionszeichnung des Herzschrittmachers aus Figur 1;
- Fig. 3:: ein Blockschaltbild des Herzschrittmachers aus Figuren 1 und 2;
- Fig. 4:: die Anordnung des Herzschrittmachers aus den Figuren 1 und 2 als AAI-Herzschrittmacher im rechten Herzohr;
- Fig. 5:: die Anordnung des Herzschrittmachers aus den Figuren 1 und 2 als VVI-Herzschrittmacher im rechten Ventrikel;
- Fig. 6:: ein von zwei implantierten Herzschrittmachern gebildetes Zweikammer-Schrittmachersystem:
- Fig. 7:: das System aus Figur 6 im nicht-implantierten Zustand;
- Fig.8:: das System aus Figuren 6 und 7 mit einer drahtgebundenen statt einer drahtlosen bidirektionalen telemetrischen Verbindung;
- Fig. 9:: ein Blockschaltbild des von zwei Herzschrittmachern 10A und 10V gebildeten Zweikammer-Schrittmachersystems; und
- Fig. 10:: einen alternativen intrakardial implantierbaren Zweikammer-Herzschrittmacher.

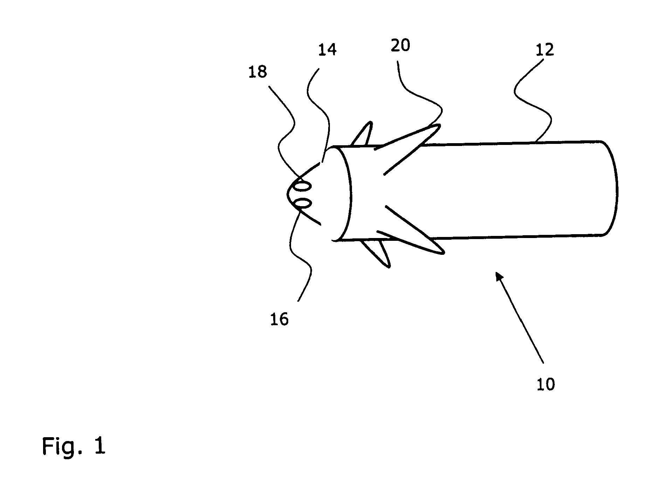

In Figur 1 ist ein Herzschrittmacher der erfindungsgemäßen Art in Form eines Einkammer-Demand-Herzschrittmachers abgebildet. Der Herzschrittmacher 10 besitzt ein längst gestrecktes, zylindrisches Gehäuse 12 mit einer halbkugelförmigen Gehäusekappe 14. An der Spitze der Gehäusekappe 14 sind zwei kleinflächige Stimulationselektroden 16 und 18 angeordnet. Um den Herzschrittmacher 10 in einer Herzkammer wie beispielsweise dem Ventrikel oder auch im Herzohr des rechten Atriums zu fixieren, sind an dem Gehäuse 12 Arretierungshaken 20 vorgesehen.

Das Gehäuse 12 ist dicht mit der Gehäusekappe 14 verbunden, so dass der von dem Gehäuse 12 und der Gehäusekappe 14 eingeschlossene Raum hermetisch abgeschlossen ist. Die Elektroden 16 und 18 sind in die Gehäusekappe 14 eingelassen und besitzen elektrisch leitende Oberflächen, die gegenüber dem übrigen Gehäuse elektrisch isoliert sind und als Pole für die Stimulation von Herzgewebe dienen.

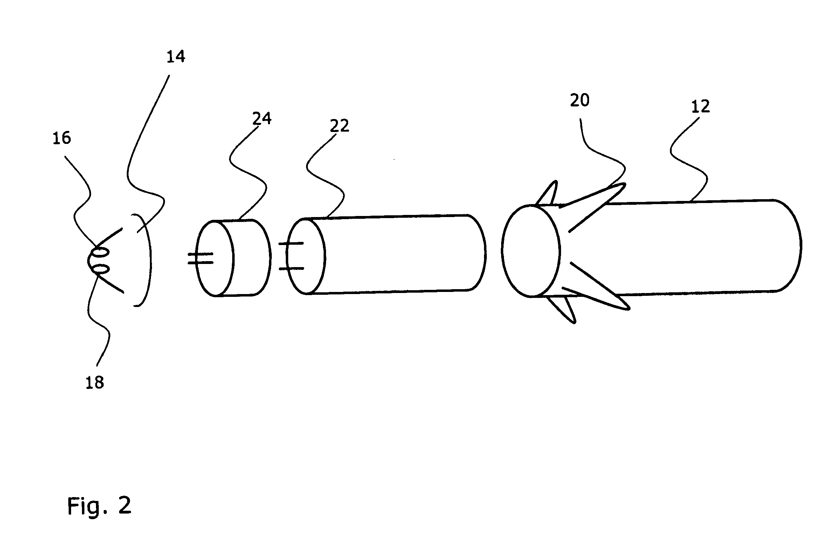

Figur 2 ist eine Explosionszeichnung des Herzschrittmachers 10 aus Figur 1 und zeigt die im Inneren des Gehäuses 12 angeordneten Bauteile, nämlich eine Batterie 22 sowie ein Elektronikmodul 24. Das Elektronikmodul 24 umfasst eine elektronische Schrittmachersteuerung sowie wenigstens einen Stimulationsimpulsgenerator und im Falle der Ausbildung des Herzschrittmachers als Demand-Schrittmacher außerdem eine Erfassungsstufe für Herzsignale. Der Stimulationsimpulsgenerator sowie die Erfassungsstufe für Herzsignale sind wechselweise jeweils mit den beiden Elektroden 16 und 18 elektrisch verbunden.

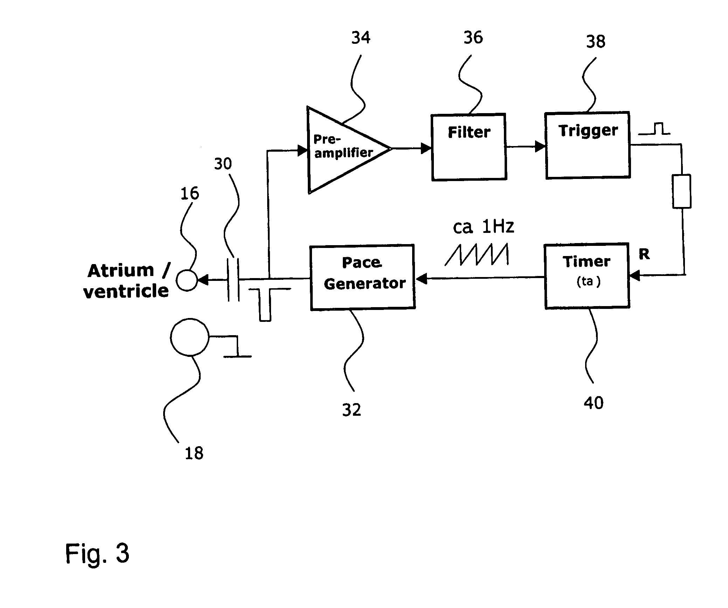

Figur 3 ist ein Blockschaltbild des Elektronikmoduls 24 eines Einkammer-Demand-Herzschrittmachers Die Elektrode 18 ist die Referenzelektrode. Die Elektrode 16 ist über einen Koppelkondensator 30 sowohl mit einem Stimulationsimpulsgenerator 32 als auch mit einer Erfassungsstufe verbunden, die einen Vorverstärker 34, einen Filter 36 sowie einen Trigger 38 umfasst. Der Trigger 38 ist mit einem Timer (Zeitgeber) 40 verbunden, der seinerseits wiederum mit dem Stimulationsimpulsgenerator 32 verbunden ist. In dieser Schaltung hat der Trigger 38 die Funktion, den Timer 40 zurückzusetzen. Falls der Timer 40 nicht zurückgesetzt wird, sondern bis zu seinem vorgegebenen Endwert weiterläuft, löst der Timer 40 über den Stimulationsimpulsgenerator 32 die Abgabe eines Stimulationsimpulses aus. Sollte die Erfassungsstufe über den Vorverstärker 34 und den Filter 36 vor Ablauf des Timers 40 eine natürliche Kammerkontraktion erfassen, gibt der Trigger 38 einen Impuls aus, der den Timer 40 zurücksetzt und auf diese Weise verhindert dass der Timer 40 ausläuft und einen Stimulationsimpuls auslöst. Werden keine natürlichen Kammerkontraktionen erfasst, läuft der Timer regelmäßig aus und setzt sich dann selbst zurück, so dass er Stimulationsimpulse mit einer eingestellten Stimulationsrate abgibt. Die Stimulationsrate kann über eine in Figur 3 nicht weiter dargestellte Schrittmachersteuerung beispielsweise telemetrisch vorgegeben werden. Die Stimulationsrate kann aber auch mit Hilfe eines physiologischen Sensors an die physiologischen Bedürfnisse des Patienten angepasst werden. Auf diese Weise ergibt sich ein ratenadaptiver Schrittmacher.

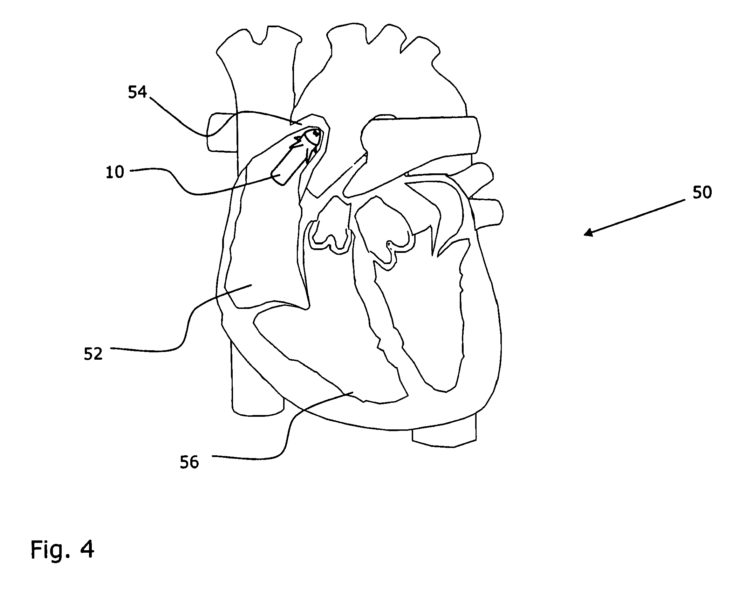

Figur 4 zeigt die Anordnung des Schrittmachers aus den Figuren 1 und 2 im vom rechten Atrium 52 eines menschlichen Herzens 50 abzweigenden rechten Herzohrs 54. In dieser Form arbeitet der Herzschrittmacher 10 als AAI-Schrittmacher.

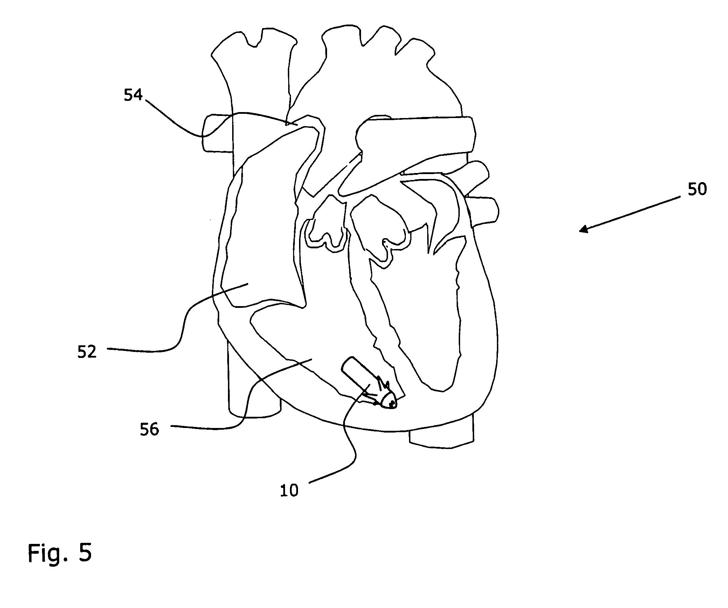

Wie Figur 5 zeigt, kann der Herzschrittmacher 10 aber auch im rechten Ventrikel 56 des Herzens 50 angeordnet sein und arbeitet dann als VVI-Schrittmacher.

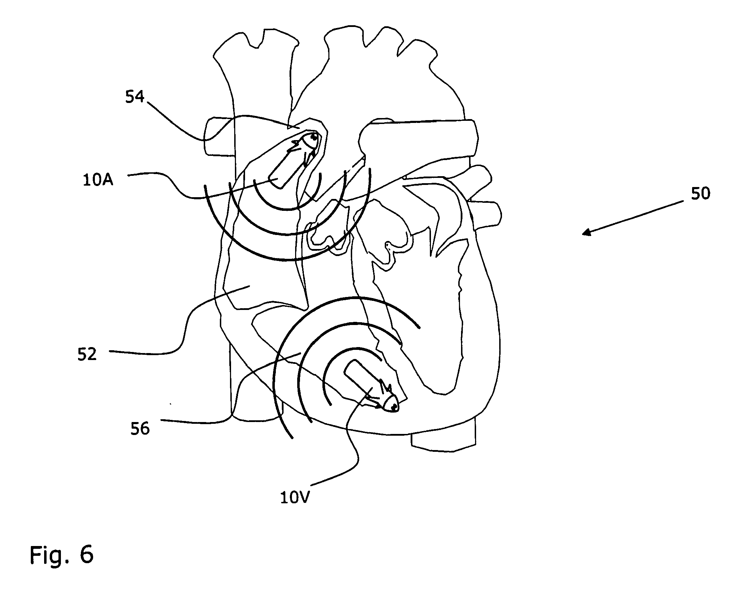

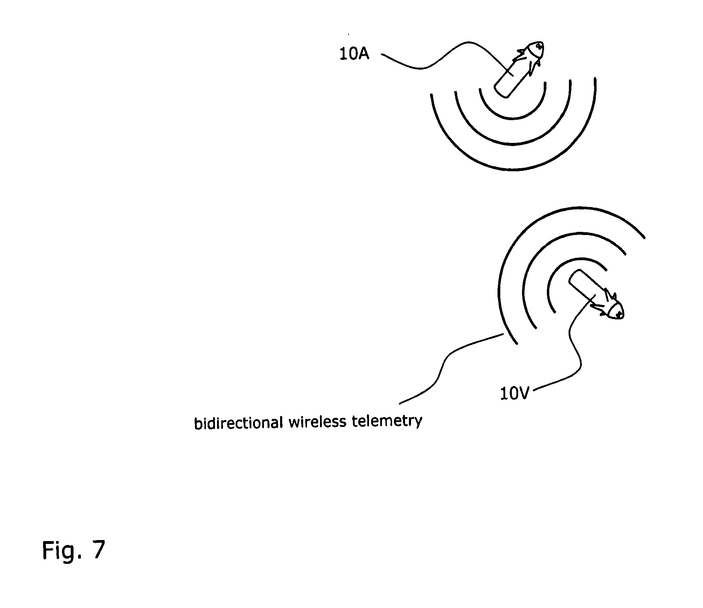

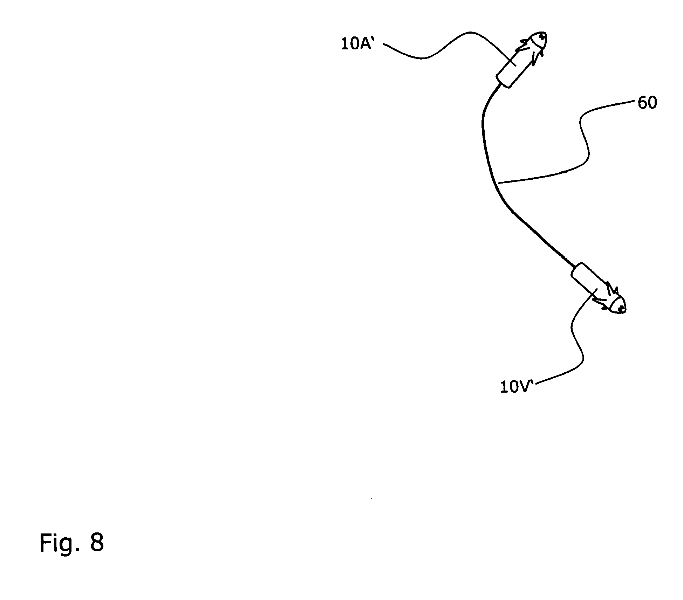

Wie zuvor bereits erwähnt, kann das Elektronikmodul 24 eines jeweiligen Schrittmachers eine drahtgebundene oder besser noch drahtlose Schnittstelle zu einem oder mehr weiteren Schrittmacher der gleichen Art besitzen, so dass zwei oder mehr Schrittmacher gemeinsam ein Mehrkammer-Schrittmachersystem bilden können, wie es in Figur 6 bis 8 abgebildet ist. In Figur 6 ist ein Zweikammersystem abgebildet. Ein erster Schrittmacher 10A ist im rechten Herzohr des rechten Atriums 52 angeordnet, während ein zweiter Herzschrittmacher 10V im rechten Ventrikel 56 des Herzens 50 angeordnet ist. Das von zwei Herzschrittmachern 10A und 10V mit jeweils drahtloser Schnittstelle gebildete System ist in Figur 7 noch einmal abgebildet. Figur 8 zeigt eine weniger bevorzugte Variante, bei der die Herzschrittmacher 10A' und 10V' über einen Draht 60 und entsprechende drahtgebundene Schnittstellen miteinander verbunden sind.

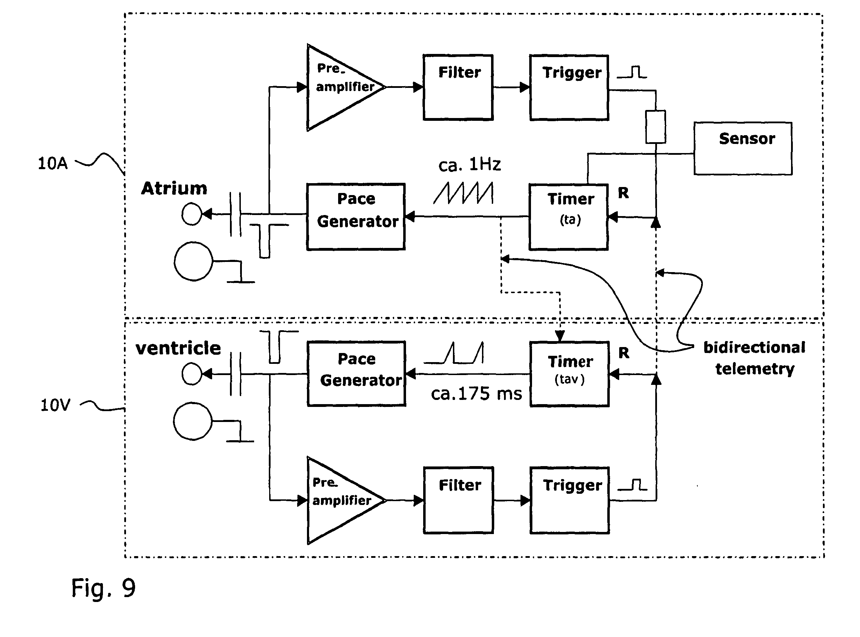

Figur 9 zeigt ein Blockschaltbild des von wie Herzschrittmachern 10A und 10V gebildeten Zweikammer-Schrittmachersystems. Das Blockschaltbild ist das Blockschaltbild eines üblichen Zweikammer-Herzschrittmachers, der jedoch im vorliegenden Fall auf zwei Gehäuse aufgeteilt ist. Diese Gehäuse sind durch eine jeweilige strichpunktierte Linie angedeutet. Der obere Teil des Blockschaltbildes repräsentiert dabei denjenigen Teil, der dem im Atrium zu platzierenden Herzschrittmacher 10A zuzuordnen ist, während der untere Teil des Blockschaltbildes die Schaltung des im Ventrikel zu platzierenden Herzschrittmachers 10V repräsentiert. Die Schaltung des Herzschrittmachers 10A und die Schaltung des Herzschrittmachers 10V sind über entsprechende drahtlose Schnittstellen und eine bidirektionale, drahtlose Telemetrie miteinander verbunden. Dies ist in dem Blockschaltbild durch gestrichelte Linien dargestellt. Auf diese Weise kann ein AV-Timer im ventrikulären Herzschrittmacher 10V durch das Erfassen eines atrialen Ereignisses oder durch die Abgabe eines atrialen Stimulationsimpulses ausgelöst werden, während umgekehrt das Erfassen eines ventrikulären Ereignisses den Timer für die Abgabe des atrialen Stimulationsimpulses auslöst. Weiterhin sei darauf hingewiesen, dass in Figur 9 auch derjenige Sensor für den physiologischen Bedarf des Patienten angedeutet ist, der bereits im Zusammenhang mit Figur 3 erwähnt wurde.

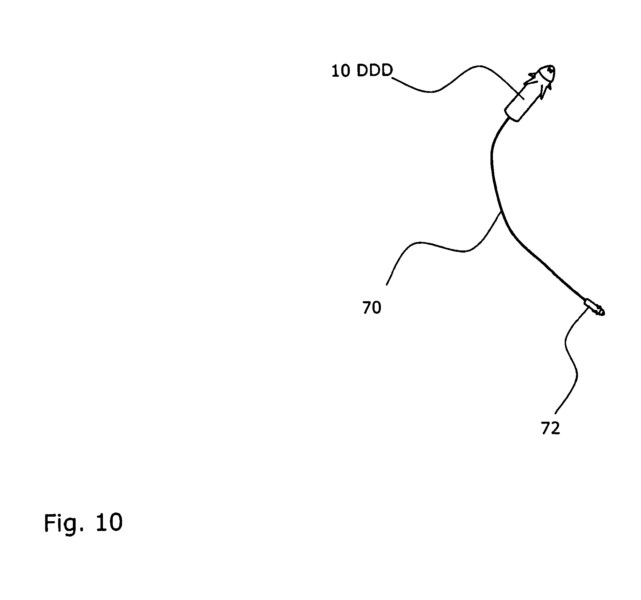

Figur 10 zeigt schließlich eine Variante eines Zweikammer-Herzschrittmachers 10DDD, bei dem die gesamte in Figur 9 dargestellte Elektronik in einem Gehäuse untergebracht ist, welches beispielsweise im Herzohr des rechten Atriums zu implantieren ist. Die Telemetrie wird dann durch eine drahtgebundene Verbindung ersetzt. An diesen Herzschrittmacher 10DDD ist eine biegeweiche Elektrodenleitung 70 befestigt, die an ihrem Ende einen bipolaren Elektrodenkopf 72 trägt, der im Apex des rechten Ventrikels zu platzieren ist. Die Elektrodenleitung 70 ist dementsprechend so bemessen, dass sie die Entfernung vom rechten Herzohr bis zum Apex des rechten Ventrikels überbrückt. Auch der Herzschrittmacher 10DDD gemäß Figur 10 ist somit vollständig im Herzen (endokardial und / oder intravaskulär) implantierbar.

## Patentansprüche

1. endokardialer oder intravaskulärer Herzschrittmacher (10) zur Stimulation einer ersten Herzkammer umfassend ein dichtes Gehäuse (12), in welchem
eine Batterie (22) und
eine mit der Batterie verbundene Schrittmachersteuerung sowie wenigstens ein Stimulationsimpulsgenerator (24)
angeordnet sind,
wobei
das Gehäuse längsgestreckt ist und
eine Länge von weniger als 70 mm sowie
eine Querschnittsfläche von weniger als 100 mm² besitzt und wenigstens zwei Elektroden (16,18) trägt,
die jeweils eine nach außen gerichtete, elektrisch leitende Oberfläche aufweisen und
als Stimulationselektroden ausgebildet sowie wenigstens zeitweise mit dem Stimulationsimpulsgenerator elektrisch über eine im Inneren des Gehäuses angeordnete elektrische Verbindung verbunden sind
**dadurch gekennzeichnet, dass** der Herzschrittmacher eine drahtlose Schnittstelle aufweist, die mit der Schrittmachersteuerung verbunden und ausgebildet ist, mit einer entsprechenden Schnittstelle eines zweiten Herschrittmachers zur Stimulation einer zweiten Herzkammer Daten bezüglich der Zeitpunkte in der jeweiligen Herzkammer auftretender Ereignisse auszutauschen, so dass die Schrittmachersteuerung des jeweils einen Herzschrittmachers auf Ereignisse in der jeweils anderen Herzkammer ansprechen kann.

2. Herzschrittmacher nach Anspruch 1, **dadurch gekennzeichnet, dass** das Querschnittsmaß des Gehäuses maximal 12 mm beträgt.

3. Herzschrittmacher nach Anspruch 1 oder 2, **dadurch gekennzeichnet, dass** das Gehäusevolumen maximal 7 cm³ beträgt.

4. Herzschrittmacher nach einem der Ansprüche 1 bis 3, **dadurch gekennzeichnet, dass** das Gehäuse wenigstens annähernd kreisrunden Querschnitt besitzt.

5. Herzschrittmacher nach Anspruch 4, **dadurch gekennzeichnet, dass** das Gehäuse über den größten Teil seiner Länge zylindrisch geformt ist.

6. Herzschrittmacher nach einem der Ansprüche 1 bis 5, **dadurch gekennzeichnet, dass** der Herzschrittmacher zur Implantation im Herzen ausgebildet ist.

7. Herzschrittmacher nach Anspruch 6, **dadurch gekennzeichnet, dass** der Herzschrittmacher zur Implantation im Herzohr ausgebildet ist.

8. Herzschrittmacher nach Anspruch 6, **dadurch gekennzeichnet, dass** der Herzschrittmacher zur Implantation im Ventrikel ausgebildet ist.

9. Herzschrittmacher nach einem der Ansprüche 1 bis 8, **dadurch gekennzeichnet, dass** wenigstens eine erste der Elektroden eine Fläche von weniger als 5mm² besitzt.

10. Herzschrittmacher nach Anspruch 9, **dadurch gekennzeichnet, dass** die erste Elektrode an einem Längsende des Gehäuses angeordnet ist.

11. Herzschrittmacher nach einem der Ansprüche 1 bis 8, **dadurch gekennzeichnet, dass** die Elektroden als Ringelektroden ausgebildet sind, die jeweils um den Umfang des Gehäuses umlaufen.

12. Herzschrittmacher nach einem der Ansprüche 1 bis 11, **dadurch gekennzeichnet, dass** im Gehäuse eine Erfassungsstufe für Herzsignale angeordnet ist und die Elektroden zusätzlich als Abfühlelektroden ausgebildet und wenigstens zeitweise mit der Erfassungsstufe verbunden sind.

13. Herzschrittmacher nach einem der Ansprüche 1 bis 12, **dadurch gekennzeichnet, dass** der Herzschrittmacher einen Telemetriesender und -empfänger besitzt, der mit der Schrittmachersteuerung verbunden und zum Aussenden von Betriebsdaten und physiologischen Daten sowie zum Empfangen von Programmier- und/oder Steuerbefehlen und oder Betriebesparametern ausgebildet ist.

14. Herzschrittmacher nach einem der Ansprüche 1 bis 13, **gekennzeichnet durch** eine an das Gehäuse angeschlossene, biegeweiche Elektrodenleitung, welche wenigstens eine Stimulationselektrode an ihrem freien Ende oder in der Nähe ihres freien Endes trägt und einen elektrischen Leiter besitzt, der die Stimulationselektrode zumindest zeitweise mit dem Stimulationsimpulsgenerator verbindet.

15. Herzschrittmacher nach Anspruch 4, **dadurch gekennzeichnet, dass** die Batterie einen wenigsten annähernd kreisrunden Querschnitt besitzt.

16. Herzschrittmacher nach Anspruch 15, **dadurch gekennzeichnet, dass** die Batterie gewickelte Elektroden besitzt.

17. Herzschrittmacher nach einem der Ansprüche 1 bis 16, **dadurch gekennzeichnet, dass** die Batterie ein Kapazität zwischen 0,25 Ah und 0,75 Ah besitzt.

18. Herzschrittmacher nach einem der Ansprüche 1 bis 17, **dadurch gekennzeichnet, dass** die Batterie ein Volumen zwischen 1 cm³ und 3 cm³ einnimmt.

19. Herzschrittmacher nach einem der Ansprüche 1 bis 18, **dadurch gekennzeichnet, dass** das Gehäuse von Metall-Gehäuseelementen gebildet ist, die miteinander verschweißt und wenigstens gegenüber einer ersten Elektrode isoliert sind.

20. Herzschrittmacher nach einem der Ansprüche 1 bis 18, **dadurch gekennzeichnet, dass** das Gehäuse von Gehäuseelementen aus elektrisch isolierendem Material wie Kunststoff oder Keramik gebildet ist.

21. Herzschrittmacher nach Anspruch 19 oder 20, **dadurch gekennzeichnet, dass** wenigstens eines der Gehäuseelemente röhrenförmig ausgebildet ist.

22. System aus zwei oder mehr Herzschrittmachern (10A, 10V) mit jeweils einem dichten Gehäuse (12), in welchem
eine Batterie (22) und
eine mit der Batterie verbundene Schrittmachersteuerung sowie wenigstens ein Stimulationsimpulsgenerator (24)
angeordnet sind,
wobei
das Gehäuse längsgestreckt ist und
eine Länge von weniger als 70 mm sowie
eine Querschnittsfläche von weniger als 100 mm² besitzt und wenigstens zwei Elektroden (16,18) trägt,
die jeweils eine nach außen gerichtete, elektrisch leitende Oberfläche aufweisen und
als Stimulationselektroden ausgebildet sowie wenigstens zeitweise mit dem Stimulationsimpulsgenerator elektrisch über eine im Inneren des Gehäuses angeordnete elektrische Verbindung verbunden sind
**dadurch gekennzeichnet, dass**
jeder Herzschrittmacher jeweils eine drahtgebundene oder vorzugsweise eine drahtlose Schnittstelle aufweist, die mit der jeweiligen Schrittmachersteuerung verbunden und ausgebildet ist, mit einer entsprechenden Schnittstelle eines zweiten Herzschrittmachers Daten auszutauschen, und
wobei die Herzschrittmacher zusammen ein Mehrkammerschrittmachersystem bilden.

## Claims

1. An endocardial or intravascular cardiac pacemaker (10) for stimulating a first chamber of the heart, comprising a sealed housing (12), in which
a battery (22) and
a pacemaker controller connected to the battery and
at least one stimulation pulse generator (24) are situated,
the housing being elongate and
having a length of less than 70 mm and
a cross-sectional area of less than 100 mm² and
carrying at least two electrodes (16, 18),
each of which has an outwardly directed, electrically conductive surface and
is implemented as a stimulation electrode and
is electrically connected at least sometimes to the stimulation pulse generator via an electrical connection positioned in the interior of the housing,
**characterized in that** the cardiac pacemaker has a wireless interface which is connected to the pacemaker controller and is implemented to exchange data with a corresponding interface of a second cardiac pacemaker for stimulating a second chamber of the heart with to the times of events occurring in the particular chamber of the heart, so that the pacemaker controller of the particular one cardiac pacemaker may respond to events in the particular other chamber of the heart.

2. The cardiac pacemaker according to Claim 1, **characterized in that** the cross-sectional dimension of the housing is at most 12 mm.

3. The cardiac pacemaker according to Claim 1 or 2, **characterized in that** the housing volume is at most 7 cm³.

4. The cardiac pacemaker according to one of Claims 1 through 3, **characterized in that** the housing has an at least approximately circular cross-section.

5. The cardiac pacemaker according to Claim 4, **characterized in that** the housing is cylindrically shaped over the largest part of its length.

6. The cardiac pacemaker according to one of Claims 1 through 5, **characterized in that** the cardiac pacemaker is implemented for implantation in the heart.

7. The cardiac pacemaker according to Claim 6, **characterized in that** the cardiac pacemaker is implemented for implantation in the auricle of the heart.

8. The cardiac pacemaker according to Claim 6, **characterized in that** the cardiac pacemaker is implemented for implantation in the ventricle.

9. The cardiac pacemaker according to one of Claims 1 through 8, **characterized in that** at least one first electrode of the electrodes has an area of less than 5 mm².

10. The cardiac pacemaker according to Claim 9, **characterized in that** the first electrode is positioned on a longitudinal end of the housing.

11. The cardiac pacemaker according to one of Claims 1 through 8, **characterized in that** the electrodes are implemented as annular electrodes, each of which runs around the periphery of the housing.

12. The cardiac pacemaker according to one of Claims 1 through 11, **characterized in that** a detection stage for cardiac signals is positioned in the housing and the electrodes are additionally implemented as sensing electrodes and are connected at least sometimes to the detection stage.

13. The cardiac pacemaker according to one of Claims 1 through 12, **characterized in that** the cardiac pacemaker has a telemetry transceiver, which is connected to the pacemaker controller and is implemented for transmitting operational data and physiological data and for receiving programming and/or control commands and/or operational parameters.

14. The cardiac pacemaker according to one of Claims 1 through 13, **characterized by** a flexible electrode line, connected to the housing, which carries at least one stimulation electrode on its free end or in proximity to its free end and has an electrical conductor which connects the stimulation electrode at least sometimes to the stimulation pulse generator.

15. The cardiac pacemaker according to Claim 4, **characterized in that** the battery has an at least approximately circular cross-section.

16. The cardiac pacemaker according to Claim 15, **characterized in that** the battery has coiled electrodes.

17. The cardiac pacemaker according to one of Claims 1 through 16, **characterized in that** the battery has a capacitance between 0.25 Ah and 0.75 Ah.

18. The cardiac pacemaker according to one of Claims 1 through 17, **characterized in that** the battery occupies a volume between 1 cm³ and 3 cm³.

19. The cardiac pacemaker according to one of Claims 1 through 18, **characterized in that** the housing is formed by metal housing elements which are welded to one another and are insulated at least in relation to a first electrode.

20. The cardiac pacemaker according to one of Claims 1 through 18, **characterized in that** the housing is formed by housing elements made of electrically insulating materials such as plastic or ceramic.

21. The cardiac pacemaker according to Claim 19 or 20, **characterized in that** at least one of the housing elements is implemented as tubular.

22. A system made of two or more cardiac pacemakers (10A, 10V), each having a sealed housing (12), in which
a battery (12) and
a pacemaker controller connected to the battery as well as at least one stimulation pulse generator (24) are situated,
the housing being elongate and
having a length of less than 70 mm and
a cross-sectional area of less than 100 mm² and carrying at least two electrodes (16, 18),
each of which has an outwardly directed, electrically conductive surface, and
is implemented as a stimulation electrode and
is at least sometimes electrically connected to the stimulation pulse generator via an electrical connection situated in the interior of the housing
**characterized in that** each cardiac pacemaker has a wired or preferably a wireless interface, which is connected to the particular pacemaker controller and is implemented to exchange data with a corresponding interface of a second cardiac pacemaker, and
the cardiac pacemakers together forming a multichamber pacemaker system.

## Revendications

1. Pacemaker '10) endocardiaque ou intravasculaire pour la stimulation d'un premier ventricule comprenant un boîtier (12) étanche dans lequel sont disposées
une batterie (22) et
une commande de pacemaker reliée à la batterie ainsi qu'au moins un générateur d'impulsion de stimulation (24),
le boîtier étant étiré en longueur et présentant une longueur de moins de 70 mm ainsi qu'une surface de section de moins de 100 mm² et portant au moins deux électrodes (16, 18), qui présentent chacune une surface dirigée vers l'extérieur et électro-conductrice et sont réalisées comme des électrodes de stimulation et sont reliées au moins temporairement au générateur d'impulsion de stimulation de façon électrique par une liaison électrique disposée à l'intérieur du boîtier,
**caractérisé en ce que** le pacemaker présente une interface sans fil, qui est reliée avec la commande de pacemaker, et conçue pour échanger avec une interface appropriée d'un second pacemaker pour la stimulation d'un second ventricule des données concernant les moments d'événement apparaissant dans le ventricule respectif, de sorte que la commande de pacemaker du respectivement un pacemaker peut réagir à des événements dans l'autre ventricule respectif.

2. Pacemaker selon la revendication 1, **caractérisé en ce que** la dimension de la section du boîtier est au maximum de 12 mm.

3. Pacemaker selon la revendication 1 ou 2, **caractérisé en ce que** le volume de boîtier est au maximum de 7 cm³.

4. Pacemaker selon l'une quelconque des revendications 1 à 3, **caractérisé en ce que** le boîtier présente une section au moins approximativement circulaire.

5. Pacemaker selon la revendication 4, **caractérisé en ce que** le boîtier est formé de façon cylindrique sur la majeure partie de sa longueur.

6. Pacemaker selon l'une quelconque des revendications 1 à 5, **caractérisé en ce que** le pacemaker est conçu pour l'implantation dans le coeur.

7. Pacemaker selon la revendication 6, **caractérisé en ce que** le pacemaker est conçu pour l'implantation dans l'oreillette.

8. Pacemaker selon la revendication 6, **caractérisé en ce que** le pacemaker est conçu pour l'implantation dans le ventricule.

9. Pacemaker selon l'une quelconque des revendications 1 à 8, **caractérisé en ce qu'**au moins une première des électrodes présente une surface de moins de 5 mm².

10. Pacemaker selon la revendication 9, **caractérisé en ce que** la première électrode est disposée sur une extrémité longitudinale du boîtier.

11. Pacemaker selon l'une quelconque des revendications 1 à 8, **caractérisé en ce que** les électrodes sont conçues comme des électrodes annulaires qui tournent respectivement autour du périmètre du boîtier.

12. Pacemaker selon l'une quelconque des revendications 1 à 11, **caractérisé en ce que** un niveau d'enregistrement pour des signaux cardiaques est disposé dans le boîtier et les électrodes sont réalisées en supplément comme des électrodes de palpation et sont reliées au moins par moments au niveau d'enregistrement.

13. Pacemaker selon l'une quelconque des revendications 1 à 12, **caractérisé en ce que** le pacemaker présente un émetteur et récepteur de télémétrie, qui est relié à la commande de pacemaker et est conçu pour l'envoi de données de service et de données physiologiques ainsi que pour la réception d'instructions de programmation et/ou de commandes et/ou de paramètres de service.

14. Pacemaker selon l'une quelconque des revendications 1 à 13, **caractérisé par** une ligne d'électrodes souple en flexion et raccordée au boîtier, qui porte au moins une électrode de stimulation sur son extrémité libre ou à proximité de son extrémité libre et présente un conducteur électrique qui relie l'électrode de stimulation au moins par moments au générateur d'impulsion de stimulation.

15. Pacemaker selon la revendication 4, **caractérisé en ce que** la batterie présente une section au moins approximativement circulaire.

16. Pacemaker, selon la revendication 15, **caractérisé en ce que** la batterie présente des électrodes enroulées.

17. Pacemaker selon l'une quelconque des revendications 1 à 16, **caractérisé en ce que** la batterie présente une capacité comprise entre 0,25 Ah et 0,75 Ah.

18. Pacemaker selon l'une quelconque des revendications 1 à 17, **caractérisé en ce que** la batterie occupe un volume compris entre 1 cm³ et 3 cm³.

19. Pacemaker selon l'une quelconque des revendications 1 à 18, **caractérisé en ce que** le boîtier est formé d'éléments de boîtier en métal, qui sont soudés entre eux et sont isolés au moins par rapport à une première électrode.

20. Pacemaker selon l'une quelconque des revendications 1 à 18, **caractérisé en ce que** le boîtier est formé d'éléments de boîtier à base de matériau électro-isolant comme du plastique ou de la céramique.

21. Pacemaker selon la revendication 19 ou 20, **caractérisé en ce que** au moins l'un des éléments de boîtier est réalisé avec une forme de tube.

22. Système constitué de deux ou plus de deux pacemakers (10A, 10V) comprenant chacun un boîtier (12) étanche dans lequel sont disposés une batterie (22) et une commande de pacemaker reliée à la batterie ainsi qu'au moins un générateur d'impulsion de stimulation (24),
le boîtier étant étiré en longueur et présentant une longueur de moins de 70 mm ainsi qu'une surface de section de moins de 100 mm² et portant au moins deux électrodes (16, 18), qui présentent chacune une surface dirigée vers le haut et électro-conductrice et sont réalisées comme des électrodes de stimulation et sont reliées au moins temporairement au générateur d'impulsion de stimulation au plan électrique par une liaison électrique disposée à l'intérieur du boîtier,
**caractérisé en ce que** chaque pacemaker présente respectivement une interface reliée par fil ou de préférence une interface sans fil, qui est reliée à la commande d'interface respective et est conçue pour échanger des données avec une interface appropriée d'un second pacemaker, et
les pacemakers formant ensemble un système de pacemaker à plusieurs chambres.
